# EUROPEAN PATENT APPLICATION

(11) **EP 3 834 731 A1**
(43) Date of publication of application: **16.06.2021**
(21) Application number: 19215656.0
(22) Date of filing: 12.12.2019
(51) Int. Cl.: A61B 6/00, G21K 1/10

(54) **COMBINED K-EDGE FILTERS FOR DOSE REDUCTION IN X-RAY IMAGING**

(71) Applicant: Koninklijke Philips N.V., 5656 AG Eindhoven (NL)
(72) Inventor: THRANL, Axel, 5656 AE Eindhoven (NL)
(74) Representative: Philips Intellectual Property & Standards

(57) **Abstract**

An X-ray filter (102) for an X-ray imaging system (100) is proposed. The X-ray filter is configured to provide a high attenuation for photon energies below a cutoff energy and a low attenuation for photon energies above the cutoff energy. The X-ray filter comprises first and second substances, wherein the first substance is a chemical element or a compound comprising atoms having a first maximum k-edge energy that is smaller than the cutoff energy, and wherein the second substance is a chemical element or a compound comprising atoms having a second maximum k-edge energy that is smaller than the first maximum k-edge energy. The second substance of the X-ray filter is configured to improve the attenuation of the X-ray filter in particular for photon energies directly below the first maximum k-edge energy.

## Description

### FIELD OF THE INVENTION

The invention relates to an X-ray filter for dose reduction in X-ray imaging, the filter being configured to provide a high attenuation for photon energies below a cutoff energy and a low attenuation for photon energies above the cutoff energy. The invention further relates to an X-ray imaging system comprising an X-ray filter for dose reduction and a method of operating an X-ray imaging system comprising such an X-ray filter.

### BACKGROUND OF THE INVENTION

X-ray imaging systems are utilized in a number of applications such as medical diagnostics, airport security, material analysis and others. For example, in medical applications, an X-ray tube and an X-ray imaging detector are arranged on opposite sides of a patient. The X-ray tube generates a beam of X-rays. The photons of the X-ray beam are partially absorbed by the patient's body. Thereby, bones absorb more photons per volume as compared to soft tissue. The photons passing through the patient's body are then received by the X-ray imaging detector, which generates a shadow image of the patient's anatomy. The resulting image is a two-dimensional projection of the three-dimensional structure of the patient's body.

The X-ray imaging detector may comprise an X-ray converter and a detector plate. The X-ray converter converts X-ray radiation into electrical charges. This conversion may be direct or indirect. For example, amorphous selenium may be used for converting X-ray radiation directly into electrical charges. Alternatively, an indirect detector may comprise a scintillator for converting X-ray radiation into visible light, which may then be converted into electrical charges by means of photodiodes. The electrical charges generated by the X-ray converter may be collected by the detector plate. Thereto, the detector plate may comprise an array such as a uniform rectangular array of thin film transistors (TFTs). For example, each TFT may be connected to a photodiode for collecting the charges generated by this photodiode. The detector plate may further comprise electronics for reading out the electrical charges. These readout electronics may further be configured to convert the electrical charges into digital pixel values. As a result, the detector plate may generate a digital image.

In X-ray imaging, the X-ray beam generated by the X-ray tube is usually filtered with an X-ray filter in order to minimize the intensities of low energy parts of the X-ray spectrum. The low energy photons are filtered out, since these photons would otherwise be absorbed within the object to be imaged, so these photons would not contribute to the image, but just to the applied X-ray dose (which is preferably minimized). An optimal filter provides a high attenuation up to a cutoff photon energy and a low attenuation for higher photon energies.

### SUMMARY OF THE INVENTION

Conventional X-ray filters are made of a chemical element having a k-edge energy below the cutoff photon energy. For example, such X-ray filters are made of aluminum, copper, or titanium. X-ray filters made of a single material may provide a strong attenuation in a photon energy range directly above the k-edge energy of the filter material. However, the attenuation may be insufficient for photon energies below the k-edge energy of the filter material. Hence, it may be desirable to improve the attenuation for photon energies below the k-edge energy of the filter material.

This is achieved by the subject matter of the independent claims, wherein further embodiments are incorporated in the dependent claims and the following description. It should be noted that any feature, element, and/or function of the X-ray filter, as described in the following, equally applies to the X-ray imaging system, as described in the following, and vice versa.

According to the present invention, an X-ray filter for an X-ray imaging system is presented. The X-ray filter is configured to attenuate X-ray photons having energies below a cutoff energy and to let pass X-ray photons having energies above the cutoff energy, wherein the X-ray filter comprises first and second substances, wherein the first substance is a chemical element or a compound comprising atoms having a first maximum k-edge energy that is smaller than the cutoff energy, and wherein the second substance is a chemical element or a compound comprising atoms having a second maximum k-edge energy that is smaller than the first maximum k-edge energy.

The X-ray filter may be utilized in an X-ray imaging system, which may comprise, besides the X-ray filter, an X-ray tube and an X-ray detector. The X-ray filter may be arranged between the X-ray tube and the X-ray detector to attenuate low-energy X-ray radiation emitted by the X-ray tube. More specifically, the X-ray filter may be arranged between the X-ray tube and the object to be imaged, and the X-ray filter may be configured to attenuate low energy X-ray radiation, which would otherwise be absorbed by the object to be imaged without contributing to the image generated by the X-ray detector.

The X-ray filter may be a thin plate. The plate may be arranged orthogonal to the imaging direction, i.e., orthogonal to the direction of the X-ray beam emitted by the X-ray tube. The thickness of the plate in the imaging direction may be small as compared to the dimensions in the orthogonal directions. Alternatively, the X-ray filter may have a curved design, wherein the thickness of the X-ray filter may be small as compared to the dimensions of the curved surface.

The X-ray filter is configured to attenuate X-ray photons having energies below a cutoff energy and to let pass X-ray photons having energies above the cutoff energy. Hence, the X-ray filter is configured to provide a high-pass characteristic. The cutoff energy may be chosen depending on the imaging application and the spectrum of the X-ray radiation provided by the X-ray tube. For example in mammography, the cutoff energy may lie between 15 and 30 keV. In other medical imaging applications, the cutoff energy may take larger values, for example values above 40 keV.

The X-ray filter comprises first and second substances. Thereby, the first substance is a chemical element or a compound, wherein the atoms of the first substance have a first maximum k-edge energy that is smaller than the cutoff energy. For example, the first substance may be a metal or a metal oxide. In case of a metal oxide, the first maximum k-edge energy may be equal to the k-edge energy of the metal atoms. Similarly, the second substance is a chemical element or a compound, wherein the atoms of the second substance have a second maximum k-edge energy, which is smaller than the first maximum k-edge energy of the first substance. When the first/second substance is a compound, the first/second maximum k-edge energy corresponds to the k-edge energy of the atom in the compound having the largest atomic number. For the sake of simplicity, we also refer to the first/second maximum k-edge energy when the first/second substance is a chemical element, i.e., when the first/second substance comprises only a single type of atoms, i.e., when the first/second substance comprises only a single k-edge energy.

The first substance may provide a strong attenuation for photon energies directly above its first maximum k-edge energy. The filter attenuation may be dominated by the first substance for photon energies above the first maximum k-edge energy. At the same time, the first substance may provide an insufficient attenuation for photon energies below the first maximum k-edge energy. The second substance may provide an improved, i.e., stronger attenuation of X-ray photons, in particular for photon energies below the first maximum k-edge energy.

The X-ray filter may comprise first and second portions, wherein the first portions may comprise the first substance, and the second portions may comprise the second substance. For example, the first portions of the X-ray filter may form a first layer, and the second portions of the X-ray filter may form a second layer. Hence, the X-ray filter may comprise a first layer comprising the first substance and a second layer comprising the second substance. Alternatively, the X-ray filter may comprise a heterogeneous mixture of the first and second substances. For example, particles of the first and second substances may be uniformly mixed, for example into an epoxy resin. Alternatively, the X-ray filter may comprise a homogeneous mixture of the first and second substances such as an alloy.

Assuming, for example, an X-ray filter comprising first and second layers of first and second substances, the attenuation provided by the X-ray filter depends on the thicknesses of the layers and the linear attenuation coefficients of the first and second substances. Thereby, the linear attenuation coefficients may account for absorption and scattering, including coherent scattering. The linear attenuation coefficients depend on the photon energy of the X-ray radiation and on the filter substances. The attenuation provided by the X-ray filter may be expressed as *1 - exp(-µ₁d₁-µ₂d₂)*, where *µ₁* and *µ₂* are the linear attenuation coefficients of the first and second substances at a given photon energy, respectively, and *d₁* and *d₂* are the thicknesses of the first and second layers, respectively. The transmission of the X-ray filter is then given by *exp(-µ₁d₁-µ₂d₂).*

As noted above, the first and second substances may not be contained in separate layers of the X-ray filter. In this case, the attenuation provided by the X-ray filter may be determined based on the mass attenuation coefficients of the first and second substances, the densities of the first and second substances, and the thickness of the X-ray filter.

In an example, a ratio of the first maximum k-edge energy of the first substance divided by the second maximum k-edge energy of the second substance is less than 2.5.

As explained above, the first substance may provide a strong attenuation for photon energies directly above the first maximum k-edge energy. However, the attenuation provided by the first substance may be insufficient for photon energies below the first maximum k-edge energy.

For example, the first substance may be tin, so the first maximum k-edge energy may be 29.2 keV. For photon energies directly above the k-edge energy, tin provides an excellent attenuation of X-ray radiation. However, for photon energies ranging in particular from 20 keV to the k-edge energy at 29.2 keV, the attenuation provided by tin may be insufficient for a given imaging application.

To improve the attenuation for photon energies below the first maximum k-edge energy, the X-ray filter may further comprise a second substance having a second maximum k-edge energy smaller than the first maximum k-edge energy, so that the attenuation is improved in particular for photon energies above the second maximum k-edge energy and below the first maximum k-edge energy. The second substance may provide an excellent attenuation for photon energies directly above the second maximum k-edge energy. However, the attenuation provided by the second substance decreases for increasing photon energies above the second maximum k-edge energy. For this reason, the second maximum k-edge energy should not be too far below the first maximum k-edge energy. The second substance of the X-ray filter may therefore be chosen such that the second maximum k-edge energy is larger than 0.4 times the first maximum k-edge energy of the first substance. In other embodiments, the second substance of the X-ray filter may be chosen such that the second maximum k-edge energy is larger than 0.5, 0.6, or 0.7 times the first maximum k-edge energy of the first substance.

In another example, the first maximum k-edge energy is larger than 7 keV.

The choice of the first substance and the corresponding first maximum k-edge energy depends on the X-ray spectrum that is utilized for a given imaging application.

For example, for low-energy X-ray imaging applications, copper (k-edge at 9.0 keV) may be combined as first substance with titanium (k-edge at 5.0 keV) or iron (k-edge at 7.1 keV) as second substance, so the first maximum k-edge energy may be larger than or equal to 9 keV.

In other X-ray imaging applications such as mammography, yttrium (k-edge at 17.0 keV), zirconium (k-edge at 18.0 keV), niobium (k-edge at 19.0 keV), or molybdenum (k-edge at 20.0 keV) may be combined as first substances with copper (k-edge at 9.0 keV), zinc (k-edge at 9.7 keV), germanium (k-edge at 11.1 keV), or selenium (k-edge at 12.7 keV) as second substances. Thus, the first maximum k-edge energy may be larger than 15 keV.

In other X-ray imaging applications, tin (k-edge at 29.2 keV) may be combined as first substance with silver (k-edge at 25.5 keV) or molybdenum (k-edge at 20.0 keV) as second substance. Thus, the first maximum k-edge energy may be larger than 25 keV.

In high-energy X-ray imaging applications, lead (k-edge at 88.0 keV) or bismuth (k-edge at 90.5 keV) may be combined as first substances with neodymium (k-edge at 43.6 keV), erbium (k-edge at 57.5 keV), or tantalum (k-edge at 67.4 keV) as second substances. Thus, the first maximum k-edge energy may be larger than 80 keV.

In another example, a transmission of the second substance of the X-ray filter at a first photon energy is smaller than 0.5, wherein the first photon energy is smaller than the first maximum k-edge energy. In addition, the first photon energy may be larger than the second maximum k-edge energy.

In particular, the first photon energy may be slightly smaller than the first maximum k-edge energy. For example, the first photon energy may be *E₁ = 0.9 Eₖ₁* + *0.1 Eₖ₂*, where *Eₖ₁* and *Eₖ₂* denote the first and second maximum k-edge energies, respectively. Larger or smaller values of the first photon energy are possible as well. The first substance of the X-ray filter may provide a rather poor attenuation for the first photon energy. In other words, the transmission of the first substance of the X-ray filter may be rather large.

For example, the X-ray filter may comprise a first layer comprising the first substance and a second layer comprising the second substance. An X-ray beam may first propagate through the first layer of the X-ray filter before impinging onto the second layer. Assuming that the photons of the X-ray beam have energies equal to the first photon energy, the intensity of the X-ray beam after propagating through the first layer of the X-ray filter may be expressed as *I₀ exp(-µ₁(E₁)d₂)*, where *I₀* is the initial intensity of the X-ray beam when impinging onto the X-ray filter, *µ₁(E₁)* is the linear attenuation coefficient of the first substance at the first photon energy *E₁,* and *d₁* is the thickness of the first layer of the X-ray filter. Hence, the transmission of the first substance of the X-ray filter may be expressed as *exp(-µ₁(E₁)d₁).* For example, the transmission of the first substance may be larger than 5%, 10%, 20%, or 30%. After propagating through the first and second layers of the X-ray filter, the intensity of the X-ray beam may be expressed as *I₀ exp(-µ₁(E₁)d₁* - *µ₂(E₁)d₂)*, where *µ*₂*(E1)* is the linear attenuation coefficient of the second substance at the first photon energy, and *d₂* is the thickness of the second layer of the X-ray filter. Thus, the transmission of the X-ray filter may be given by *exp(-µ₁(E₁)d₁* - *µ₂(E₁)d₂).*

The second substance may provide a strong attenuation of X-ray radiation for photon energies directly above its second maximum k-edge energy. However, even at a first photon energy *E₁* directly below the first maximum k-edge energy, which may differ substantially from the second maximum k-edge energy, the second substance may provide a transmission *exp(-µ₂(E₁)d₂)* < ζ, where ζ may be, for example, 0.5, 0.25, 0.1, or 0.05. In particular, the transmission provided by the second layer may be smaller than ζ for all photon energies between the second maximum k-edge energy *Eₖ₂* and the first maximum k-edge energy *Eₖ₁.* As a result, the transmission *exp(-µ₁(E₁)d₁)* - *µ₂(E₁)d₂)* of the X-ray filter at the first photon energy may be at most a factor of *ζ* the size of the transmission *exp(-µ₁(E₁)d₁)* of the first substance, even when the first photon energy is directly below the first maximum k-edge energy.

In another example, the transmission of the X-ray filter is less than 10% for all photon energies below the first maximum k-edge energy.

In other examples, the transmission of the X-ray filter may be less than 0.5%, 1%, 2%, or 5% for all photon energies below the first maximum k-edge energy. Hence, the X-ray filter may be configured to provide a minimum attenuation of 99.5%, 99%, 98%, or 95% for all photon energies below the first maximum k-edge energy.

The X-ray filter may further be configured to provide a large transmission for higher photon energies. For example, the X-ray filter may be configured to provide a transmission of at least 40%, 60%, 80%, 90% or 95% for photon energies well-above the first maximum k-edge energy. Consequently, the X-ray filter may be configured to provide a high-pass characteristic, i.e., the X-ray filter may be radiopaque for low-energy X-ray photons and radiolucent for high-energy X-ray photons.

In another example, the X-ray filter comprises first and second layers, wherein the first layer comprises the first substance of the X-ray filter, and wherein the second layer comprises the second substance of the X-ray filter. The first substance may be or may comprise tin, and a thickness of the first layer may be less than 0.5 mm.

The first substance may be or may comprise tin, which is suitable for many medical X-ray imaging applications due to its k-edge energy at about 29.2 keV.

The X-ray filter may be arranged relative to the X-ray tube such that the X-ray beam emitted by the X-ray tube impinges onto the first layer. After propagating through the first layer, photons of the primary X-ray beam may impinge onto the second layer of the X-ray filter. At a photon energy directly below the first maximum k-edge energy of 29.2 keV, tin provides a mass attenuation coefficient of approximately *µ₁* / *ρ* = *7.76 cm²*/*g.* With a density of *ρ* = *7.31 g*/*cm³,* the transmission of a layer of tin having a thickness of *d₁* = *0.5 mm* is given by *exp(-µ₁d₁) = 5.9%.* To improve the transmission of the first layer for high photon energies above the cutoff energy, the thickness of the first layer may be reduced substantially. For example, the thickness of the first layer may be reduced to 0.4 mm, 0.3 mm, 0.2 mm, or 0.1 mm. However, such thin first layers may not provide a sufficient attenuation for photon energies directly below the first maximum k-edge energy. The second layer may be configured to improve the attenuation of the X-ray filter in particular for photon energies directly below the first maximum k-edge energy.

In another example, the first maximum k-edge energy is smaller than 30 keV.

In many medical X-ray imaging applications, the first maximum k-edge energy may be smaller than 30 keV to prevent the attenuation of X-ray radiation that is suitable for such imaging applications.

However, in other applications such as material analysis, the first maximum k-edge energy may be larger than 30 keV. In such applications, the first maximum k-edge energy may be smaller than 50 keV, 75 keV, or 100 keV.

In another example, the first substance comprises tin, and the second substance comprises molybdenum or silver.

Hence, the first maximum k-edge energy may be 29.2 keV (tin), and the second maximum k-edge energy may be 25.5 keV (silver) or 20.0 keV (molybdenum).

It is also possible that the X-ray filter comprises first, second, and third substances, wherein the first substance comprises tin, the second substance comprises molybdenum, and the third substance comprises silver. For example, the X-ray filter may comprise a first layer comprising the first substance, a second layer comprising the second substance, and a third layer comprising the third substance.

In another example, the first substance comprises molybdenum, zirconium, niobium, or yttrium, and the second substance comprises copper, zinc, germanium, selenium, cobalt, or nickel.

Hence, the first maximum k-edge energy may be 17.0 keV (yttrium), 18.0 keV (zirconium), 19.0 keV (niobium), or 20.0 keV (molybdenum). The second maximum k-edge energy may be 9.0 keV (copper), 9.7 keV (zinc), 11.1 keV (germanium), or 12.7 keV (selenium). X-ray filters with such first and second maximum k-edge energies may be suitable in particular for imaging applications such as mammography, wherein low-energy X-rays are utilized.

In another example, the X-ray filter further comprises a third substance, wherein the third substance is a chemical element or a compound comprising atoms having a third maximum k-edge energy that is smaller than the second maximum k-edge energy of the second substance.

The first and second substances of the X-ray filter may provide an insufficient attenuation for photon energies below the second maximum k-edge energy. The third substance may be configured to improve the attenuation in particular for photon energies below the second maximum k-edge energy. For this reason, the third maximum k-edge energy shall be smaller than the second maximum k-edge energy of the second substance.

The third substance is chemical element or a compound. For example, the first substance may be a metal or a metal oxide. In case of a metal oxide, the first maximum k-edge energy may be equal to the k-edge energy of the metal atoms. When the third substance is a compound, the third maximum k-edge energy corresponds to the k-edge energy of the atom in the compound having the largest atomic number. For the sake of simplicity, we also refer to the third maximum k-edge energy when the third substance is a chemical element, i.e., when the third substance comprises only a single k-edge energy.

The X-ray filter may comprise first, second, and third portions, wherein the first portions may comprise the first substance, the second portions may comprise the second substance, and the third portions may comprise the third substance. For example, the first, second, and third portions of the X-ray filter may form first, second, and third layers, respectively. Hence, the X-ray filter may comprise a first layer comprising the first substance, a second layer comprising the second substance, and a third layer comprising the third substance. Alternatively, the X-ray filter may comprise a heterogeneous mixture of the first, second, and third substances. For example, particles of the first, second, and third substances may be uniformly mixed, for example into an epoxy resin. Alternatively, the X-ray filter may comprise a homogeneous mixture of the first, second, and third substances such as an alloy.

In another example, a transmission of the third substance of the X-ray filter at a second photon energy is smaller than 0.5, wherein the second photon energy is smaller than the second maximum k-edge energy. Furthermore, the second photon energy may be larger than the third maximum k-edge energy.

In particular, the second photon energy may be slightly smaller than the second maximum k-edge energy. For example, the second photon energy may be *E₂* = *0.9 Eₖ₂* + *0.1 Eₖ₃,* where *Eₖ₃* denotes the third maximum k-edge energy. Larger or smaller values of the second photon energy are possible as well. The first and second substances of the X-ray filter may provide a poor attenuation for the second photon energy. In other words, the transmission of the combination of the first and second substances of the X-ray filter may be large for the second photon energy.

For example, the X-ray filter may comprise a first layer comprising the first substance, a second layer comprising the second substance, and a third layer comprising the third substance. The first, second, and third layers of the X-ray filter may be arranged in this order. An X-ray beam may first propagate through the first and second layers of the X-ray filter before impinging onto the third layer. Assuming that the photons of the X-ray beam have energies equal to the second photon energy, the intensity of the X-ray beam after propagating through the first and second layers of the X-ray filter may be expressed as *I₀ exp(-µ₁(E₂)d₁-µ₂(E₂)d₂)*, where *µ₁(E₂)* and *µ₂(E₂)* are the linear attenuation coefficients of the first and second substances at the second photon energy, respectively. Hence, the transmission of the first and second substances of the X-ray filter may be written as *exp(-µ₁(E₂)d₁-µ₂(E₂)d₂).* For example, the transmission of the first and second substances at the second photon energy may be larger than 5%, 10%, 20%, or 30%. After propagating through the first, second, and third layers of the X-ray filter, the intensity of the X-ray beam may be *I₀ exp(-µ₁(E₂)d₁* - *µ₂(E₂)d₂* - *µ₃(E₂)d₃)*, where *µ*₃*(E₂)* is the linear attenuation coefficient of the third substance at the second photon energy, and *d₃* is the thickness of the third layer of the X-ray filter. Thus, the transmission of the X-ray filter may be given by *exp(-µ₁(E₂)d₁* - *µ₂(E₂)d₂* - *µ₃(E₂)d₃).*

The third substance may provide a strong attenuation of X-ray radiation in particular for photon energies directly above its third maximum k-edge energy. However, even at a second photon energy *E₂* directly below the second maximum k-edge energy, which may be substantially larger than the third maximum k-edge energy, the third substance may provide a transmission *exp(-µ₃(E₂)d₃)* < *ξ*, where *ξ* may be, for example, 0.5, 0.25, 0.1, or 0.05. In particular, the transmission provided by the third layer may be smaller than *ξ* for all photon energies between the third maximum k-edge energy *Eₖ₃* and the second maximum k-edge energy *Eₖ₂.* As a result, the transmission *exp(-µ₁(E₂)d₁* - *µ₂(E₂)d₂* - *µ₃(E₂)d₃)* of the X-ray filter at the second photon energy may be smaller than a factor of *ξ* the transmission *exp(-µ₁(E₂)d₁-µ₂(E₂)d₂)* of the first and second layers of the X-ray filter, even when the second photon energy is directly below the second maximum k-edge energy.

In another example, the third substance comprises polytetrafluoroethylene, aluminum, titanium, copper, or molybdenum.

For example, the first substance of the X-ray filter may comprise tin, the second substance of the X-ray filter may comprise silver, and the third substance of the X-ray filter may comprise molybdenum.

In other examples, the third substance of the X-ray filter may be or may comprise polytetrafluoroethylene, aluminum, or titanium. Hence, the third substance may comprise or may consist of atoms having low atomic numbers.

The third substances may be configured to attenuate k-escape photons, which may be emitted by the first and second substances, and which may have energies equal to or smaller than the first or second maximum k-edge energies, respectively.

For example, the X-ray filter may comprise a first layer comprising the first substance, a second layer comprising the second substance, and a third layer comprising the third substance. The first, second, and third layers of the X-ray filter may be arranged in this order. Furthermore, the X-ray filter may be arranged relative to an X-ray tube such that photons of the X-ray beam emitted by the X-ray tube propagate, in this order, through the first, second, and third layers. In this case, the first layer may emit k-escape photons having energies equal to or smaller than the first maximum k-edge energy. These k-escape photons may be attenuated by the second and third layers of the X-ray filter. Hence, the second and third layers of the X-ray filter may be configured to prevent that k-escape photons having energies equal to or smaller than the first maximum k-edge energy reach the object to be imaged. Furthermore, the second layer may emit k-escape photons having energies equal to or smaller than the second maximum k-edge energy. These k-escape photons may be attenuated by the third layer of the X-ray filter. Hence, the third layer of the X-ray filter may be configured to prevent that k-escape photons having energies equal to or smaller than the second maximum k-edge energy reach the object to be imaged.

In other embodiments, the X-ray filter may comprise more than three substances. For example, the X-ray filter may comprise a fourth substance, wherein the fourth substance may be a chemical element or a compound comprising atoms having a fourth maximum k-edge energy that is smaller than the third maximum k-edge energy of the third substance.

In another example, the X-ray filter is configured to provide a larger transmission at a third photon energy as compared to another X-ray filter consisting of the first substance, the thicknesses of the X-ray filter and the another X-ray filter being configured such that the maximum transmission for all photon energies of a low energy range is the same for both filters. Thereby, the third photon energy may be larger than the cutoff energy. Further, the maximum of the low energy range may be smaller than the cutoff energy. Further, the maximum of the low energy range may be larger than the first maximum k-edge energy. Hence, under the constraint that the transmission of the X-ray filter and the another X-ray filter does not exceed an upper bound for all photon energies of the low energy range, the X-ray filter according to the invention may provide a steeper increase of the transmission for photon energies above the cutoff energy as compared to the another X-ray filter consisting of the first substance.

According to the present invention, also an X-ray imaging system is presented. The X-ray imaging system comprises an X-ray tube for generating an X-ray beam, and an X-ray filter according to the invention, wherein the X-ray filter may be arranged for filtering the X-ray beam.

The X-ray imaging system may be configured for medical diagnostics, airport security, material analysis, or other applications. The X-ray imaging system may further comprise an X-ray imaging detector. The X-ray imaging detector may comprise an X-ray converter and a detector plate. The X-ray converter may convert X-ray radiation into electrical charges. This conversion may be direct or indirect. The electrical charges generated by the X-ray converter may be collected by the detector plate. Thereto, the detector plate may comprise an array of TFTs. The detector plate may further comprise electronics for reading out the electrical charges. These readout electronics may further be configured to convert the electrical charges into digital values. As a result, the detector plate may generate a digital image.

The X-ray imaging system may further comprise a data processing system for processing the image generated by the detector plate. Moreover, the X-ray imaging system may comprise a control unit for synchronizing the operation of the X-ray tube and the X-ray imaging detector, and/or for controlling imaging parameters such as the tube voltage, the tube current, the integration period, etc.

The X-ray filter may be arranged between the X-ray tube and the X-ray imaging detector. In particular, the X-ray filter may be arranged between the X-ray tube and the object to be imaged. Hence, the X-ray filter may be configured to reduce the exposure of the object to X-ray radiation.

In an example, the X-ray filter of the X-ray imaging system comprises first and second layers, wherein the first layer comprises the first substance of the X-ray filter, wherein the second layer comprises the second substance of the X-ray filter, and wherein the X-ray tube, the first layer, and the second layer are arranged in this order.

The X-ray tube, the first layer of the X-ray filter, the second layer of the X-ray filter, and the object to be imaged may be arranged in this order. The X-ray beam emitted by the X-ray tube may first propagate at least in part through the first layer of the X-ray filter and subsequently, at least in part, through the second layer of the X-ray filter. After passing through the X-ray filter, the X-ray beam may impinge onto the object to be imaged.

Thereby, the first layer of the X-ray filter may emit k-escape photons having energies equal to or smaller than the first maximum k-edge energy. The second layer of the X-ray filter preferably provides a strong attenuation for k-escape photons having energies equal to or smaller than the first maximum k-edge energy. Since the second layer of the X-ray filter is arranged between the first layer and the object to be imaged, it attenuates the k-escape photons emitted by the first layer. Hence, the second layer of the X-ray filter may prevent that k-escape photons having energies equal to or smaller than the first maximum k-edge energy reach the object to be imaged.

According to the present invention, also the use of an X-ray filter according to the invention in an X-ray imaging system is presented.

Hence, an X-ray image may be generated by means of an X-ray imaging system containing an X-ray filter according to the invention. Preferably, the X-ray filter is arranged between the X-ray tube of the X-ray imaging system and the object to be imaged such that the X-ray filter filters the X-ray beam emitted by the X-ray tube before it impinges onto the object to be imaged.

According to the present invention, also a method for designing an X-ray filter is presented. The method may comprise the following steps: In a first step, a cutoff energy may be determined. In a second step, first and second substances are determined, wherein the first substance is a chemical element or a compound comprising atoms having a first maximum k-edge energy that is smaller than the cutoff energy, and wherein the second substance is a chemical element or a compound comprising atoms having a second maximum k-edge energy that is smaller than the first maximum k-edge energy. In a third step, a low energy range and an upper bound for the transmission in the low energy range may be determined. The low energy range may comprise photon energies below the cutoff energy. For example, the low energy range may comprise all photon energies up to the first maximum k-edge energy. The low energy range may also comprise photon energies above the first maximum k-edge energy. The upper bound for the transmission in the low energy range may be a constant value. Alternatively, the upper bound for the transmission in the low energy range may take different values for different photon energies. In a fourth step, a third photon energy is determined. The third photon energy may be equal to or larger than the cutoff energy. The X-ray filter may comprise first and second layers, wherein the first layer comprises the first substance and the second layer comprises the second substance. In the fifth step, thicknesses of the first and second layers may be determined such that the transmission of the X-ray filter for the third photon energy is large subject to the constraint that the transmission does not exceed the upper bound for all photon energies within the low energy range. In particular, thicknesses of the first and second layers may be determined such that the transmission of the X-ray filter for the third photon energy is maximized subject to the constraint that the transmission does not exceed the upper bound for all photon energies within the low energy range.

When the first and second substances are not contained in separate layers of the X-ray filter, the transmission of the X-ray filter may be determined based on the mass attenuation coefficients of the first and second substances, the densities of the first and second substances, and the thickness of the X-ray filter. Hence, in the fifth step, the thickness of the X-ray filter and the densities of the first and second substances may be determined such that the transmission of the X-ray filter for the third photon energy is large subject to the constraint that the transmission does not exceed the upper bound for all photon energies within the low energy range. In particular, the thickness of the X-ray filter and the densities of the first and second substances may be determined such that the transmission of the X-ray filter for the third photon energy is maximized subject to the constraint that the transmission does not exceed the upper bound for all photon energies within the low energy range.

The method may be applied similarly in case of more than two substances.

It shall be understood that the X-ray filter and the X-ray imaging system as defined in the claims have similar and/or identical preferred embodiments, in particular, as defined in the dependent claims. It shall be understood further that a preferred embodiment of the invention can also be any combination of the dependent claims with the respective independent claim.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the accompanying drawings:
Figure 1 shows schematically and exemplarily an embodiment of an X-ray imaging system.
Figure 2 shows transmission curves of several example X-ray filter configurations.

### DETAILED DESCRIPTION OF EMBODIMENTS

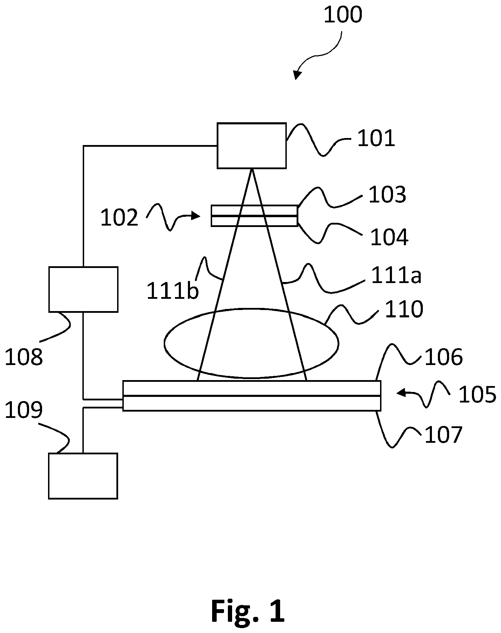

Figure 1 shows schematically and exemplarily an embodiment of an X-ray imaging system 100. The X-ray imaging system comprises an X-ray tube 101 and an X-ray filter 102. The X-ray filter may be arranged to filter an X-ray beam emitted by the X-ray tube. The shape of the X-ray beam is illustrated in Figure 1 by outermost X-rays 111a and 111b.

The X-ray filter 102 may be configured to provide a high attenuation for photon energies below a cutoff energy and a low attenuation for photon energies above the cutoff energy. Hence, the X-ray filter may be configured to provide a high-pass characteristic.

The X-ray filter may be arranged between the X-ray tube and an object 110 to be imaged. Hence, the X-ray filter may reduce the exposure of the object to X-ray radiation. More specifically, the X-ray filter may be configured to attenuate low energy X-ray radiation, which would otherwise be absorbed by the object to be imaged without contributing to the generated image.

The X-ray filter may have the shape of a plate as illustrated by Figure 1. Alternatively, the X-ray filter may have a curved design. For example, the X-ray filter may be curved such that also outermost X-rays 111a and 11b orthogonally impinge onto the X-ray filter.

The X-ray filter comprises first and second substances, wherein the first substance is a chemical element or a compound comprising atoms having a first maximum k-edge energy that is smaller than the cutoff energy, and wherein the second substance is a chemical element or a compound comprising atoms having a second maximum k-edge energy that is smaller than the first maximum k-edge energy. Optionally, the X-ray filter may comprise further substances having maximum k-edge energies below the second maximum k-edge energy. The second and further substances may be configured to provide an improved, i.e., stronger attenuation for photon energies below the first maximum k-edge energy.

The X-ray filter may comprise a first layer 103 and a second layer 104. The first layer may comprise the first substance, and the second layer may comprise the second substance. More generally, the X-ray filter may comprise first and second portions, wherein the first portions may comprise the first substance, and the second portions may comprise the second substance (not shown in Figure 1). For example, the X-ray filter may comprise a heterogeneous mixture of the first and second substances. For instance, particles of the first and second substances may be uniformly mixed, for example into an epoxy resin. Alternatively, the X-ray filter may comprise a homogeneous mixture of the first and second substances such as an alloy.

The X-ray imaging system may further comprise an X-ray imaging detector 105. The X-ray beam emitted by the X-ray tube may propagate, at least in part, through the X-ray filter and the object to be imaged before impinging onto the X-ray imaging detector. The X-ray imaging detector may comprise an X-ray converter 106 for converting X-ray radiation into electrical charges and a detector plate 107 for collecting the electrical charges generated by the X-ray converter and for generating an image.

The X-ray converter may be a direct or an indirect converter. The X-ray converter may comprise an array of conversion elements, each conversion element being configured for converting X-ray radiation into electrical charges.

The detector plate may comprise a corresponding array of detector elements for collecting the electrical charges generated by the conversion elements of the X-ray converter. Thereto, each detector element may comprise a TFT. The detector plate may further comprise readout electronics for reading out an image, wherein each pixel of the image may correspond to the electrical charges generated by one conversion element of the X-ray converter. Hence, the detector plate may be configured for generating a digital image visualizing the amount of X-ray radiation that impinged onto the X-ray converter.

The X-ray imaging system may further comprise a data processing system 109 for processing the image generated by the detector plate. Moreover, the X-ray imaging system may comprise a control unit 108 for synchronizing the operation of the X-ray tube and the X-ray imaging detector, and/or for controlling imaging parameters such as the tube voltage, the tube current, the integration period, etc.

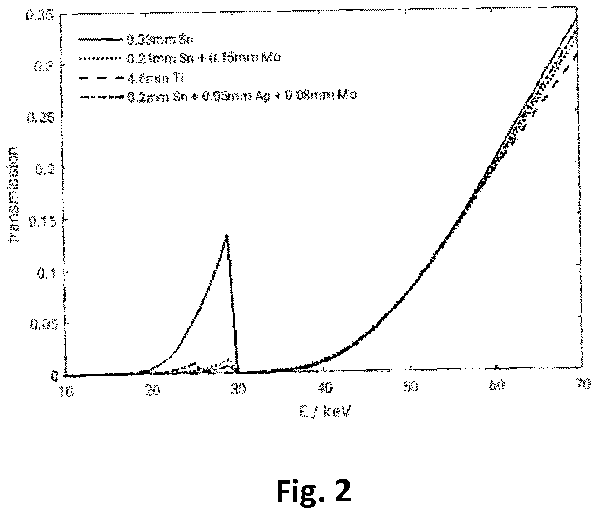

Figure 2 shows transmission curves of several example X-ray filter configurations as a function of the photon energy. Hence, the abscissa depicts the photon energy in keV, while the ordinate depicts the transmission. A cutoff energy of 50 keV may be assumed. Thus, the X-ray filters may be configured to attenuate photons having energies below this cutoff energy, and to let higher-energy photons pass. For the sake of comparison, the X-ray filters depicted in Figure 2 have been configured to provide the same transmission for the cutoff energy. The solid curve shows the transmission of an X-ray filter consisting of tin having a thickness of 0.33 mm. The solid curve is shown for the purpose of comparison. It does not correspond to an X-ray filter according to the invention, as it does not comprise first and second substances having different maximum k-edge energies. The X-ray filter consisting of tin provides a strong attenuation for photon energies directly above the k-edge energy of 29.2 keV. However, for photon energies directly below this k-edge energy, the X-ray filter provides a poor attenuation. In particular, Figure 2 shows that the transmission of the X-ray filter below its k-edge energy reaches values above 10%.

The dashed curve shows the transmission of an X-ray filter consisting of titanium having a thickness of 4.6 mm. Again, this curve does not correspond to an X-ray filter according to the invention, as it does not comprise first and second substances having different maximum k-edge energies. Titanium has a k-edge energy of 5.0 keV. Compared to the X-ray filter made of tin, the X-ray filter made of titanium provides an improved attenuation for photon energies between 20 and 29.2 keV. At the same time, Figure 2 shows that the X-ray filter made of titanium provides a worse transmission for high photon energies (above, for example, 60 keV) as compared to the X-ray filter made of tin.

The dotted curve shows the transmission for an X-ray filter according to the invention. The X-ray filter comprises a first layer made of tin and a second layer made of molybdenum. The first layer has a thickness of 0.21 mm, and the second layer has a thickness of 0.15 mm. Hence, the first maximum k-edge energy is 29.2 keV and the second maximum k-edge energy is 20.0 keV. Considering only the first layer of the X-ray filter, the transmission of this layer may reach values up to 30% for photon energies directly below the first maximum k-edge energy. However, as shown in Figure 2, the second layer made of molybdenum prevents such high transmission values. In particular, the transmission of the X-ray filter consisting of tin and molybdenum is below 2.5% for all X-ray energies below the first maximum k-edge energy. Consequently, the second layer made of molybdenum significantly improves the attenuation for low energy X-ray photons.

Compared to the X-ray filter made of tin (solid curve), the X-ray filter made of tin and molybdenum (dotted curve) provides a significantly improved attenuation for photon energies below the k-edge energy of 29.2 keV. For high photon energies (above, for example, 60 keV), the transmission provided by the X-ray filter consisting of tin and molybdenum is only slightly worse than that of the X-ray filter made of tin.

The transmission may be required to be smaller than an upper bound of, for example, 1%, 2.5%, or 5% for all photon energies of a low energy range. The low energy range may include energies up to 30 keV, 35 keV, or 40 keV. The X-ray filter consisting of tin and molybdenum may satisfy such a requirement, but the X-ray filter made of tin having a thickness of 0.33mm may not satisfy the requirement due to the poor attenuation directly below the k-edge energy at 29.2 keV. Hence, the thickness of the X-ray filter made of tin may have to be increased to satisfy the requirement of keeping the transmission below the upper bound within the low energy range. However, this increase of the thickness of the X-ray filter made of tin would result in a degradation of the transmission for high photon energies (above, for example, 60 keV). Hence, the X-ray filter made of tin and molybdenum may provide a larger transmission at a high photon energy (for example, 60 keV, 80 keV, or larger) as compared to the X-ray filter consisting of tin when both X-ray filters were configured such that the transmission does not exceed the upper bound for all photon energies of the low energy range.

Compared to the X-ray filter made of titanium (dashed curve), the X-ray filter made of tin and molybdenum (dotted curve) provides a slightly worse attenuation for photon energies below the k-edge energy of 29.2 keV. At the same time, the X-ray filter consisting of tin and molybdenum provides a substantially improved transmission as compared to the X-ray filter made of titanium for high photon energies (above, for example, 60 keV).

The dash-dotted curve shows the transmission for another X-ray filter according to the invention. This X-ray filter comprises a first layer made of tin, a second layer made of silver, and a third layer made of molybdenum. The first layer has a thickness of 0.2 mm, the second layer has a thickness of 0.05 mm, and the third layer has a thickness of 0.08 mm. Hence, the first maximum k-edge energy is 29.2 keV, the second maximum k-edge energy is 25.5 keV, and the third maximum k-edge energy is 20.0 keV. Considering only the first and second layers of the X-ray filter, the transmission of this combination of layers may be high for photon energies directly below the second photon energy. The third layer made of molybdenum prevents such high transmission values. Figure 2 shows that the transmission of the X-ray filter consisting of tin and molybdenum is below 2.5% for all X-ray energies below the first maximum k-edge energy. At the same time, the X-ray filter consisting of tin, silver, and molybdenum provides an even better transmission than the X-ray filter consisting of tin and molybdenum for high photon energies (above, for example, 60 keV).

While the invention has been illustrated and described in detail in the drawings and foregoing description, such illustrations and descriptions are to be considered illustrative or exemplary and not restrictive. In particular, the illustrated first, second, and third substances of the X-ray filter are only exemplary. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An X-ray filter (102) for an X-ray imaging system,
wherein the X-ray filter is configured to attenuate X-ray photons having energies below a cutoff energy and to let pass X-ray photons having energies above the cutoff energy,
wherein the X-ray filter comprises first and second substances,
wherein the first substance is a chemical element or a compound comprising atoms having a first maximum k-edge energy that is smaller than the cutoff energy, and
wherein the second substance is a chemical element or a compound comprising atoms having a second maximum k-edge energy that is smaller than the first maximum k-edge energy.

2. The X-ray filter (102) of claim 1,
wherein a ratio of the first maximum k-edge energy of the first substance divided by the second maximum k-edge energy of the second substance is less than 2.5.

3. The X-ray filter (102) of any of claims 1 or 2,
wherein the first maximum k-edge energy is larger than 7 keV.

4. The X-ray filter (102) of any of the preceding claims,
wherein a transmission of the second substance of the X-ray filter at a first photon energy is smaller than 0.5,
wherein the first photon energy is smaller than the first maximum k-edge energy and larger than the second maximum k-edge energy.

5. The X-ray filter (102) of any of the preceding claims,
wherein a transmission of the X-ray filter is less than 10% for all photon energies below the first maximum k-edge energy.

6. The X-ray filter (102) of any of the preceding claims,
wherein the X-ray filter comprises first and second layers,
wherein the first layer (103) comprises the first substance of the X-ray filter,
wherein the second layer (104) comprises the second substance of the X-ray filter,
wherein the first substance comprises tin, and
wherein a thickness of the first layer is less than 0.5 mm.

7. The X-ray filter (102) of any of the preceding claims,
wherein the first maximum k-edge energy is smaller than 30 keV.

8. The X-ray filter (102) of any of the preceding claims,
wherein the first substance comprises tin, and
wherein the second substance comprises molybdenum or silver.

9. The X-ray filter (102) of any of the preceding claims,
wherein the first substance comprises molybdenum, zirconium, niobium, or yttrium, and
wherein the second substance comprises copper, zinc, germanium, selenium, cobalt, or nickel.

10. The X-ray filter (102) of any of the preceding claims, further comprising a third substance,
wherein the third substance is a chemical element or a compound comprising atoms having a third maximum k-edge energy that is smaller than the second maximum k-edge energy of the second substance.

11. The X-ray filter (102) of claim 10,
wherein a transmission of the third substance of the X-ray filter at a second photon energy is smaller than 0.5, and
wherein the second photon energy is smaller than the second maximum k-edge energy and larger than the third maximum k-edge energy.

12. The X-ray filter (102) of any of claims 10 or 11,
wherein the third substance comprises polytetrafluoroethylene, aluminum, titanium, copper, or molybdenum.

13. An X-ray imaging system (100) comprising
an X-ray tube (101) for generating an X-ray beam (111a, 11 1b), and
an X-ray filter (102) according to any of the preceding claims,
wherein the X-ray filter is arranged for filtering the X-ray beam.

14. The X-ray imaging system (100) of claim 13,
wherein the X-ray filter (102) comprises first and second layers,
wherein the first layer (103) comprises the first substance of the X-ray filter,
wherein the second layer (104) comprises the second substance of the X-ray filter, and
wherein the X-ray tube (101), the first layer, and the second layer are arranged in this order.

15. Method of operating an X-ray imaging system comprising an X-ray filter according to any of the claims 1-12.
